# EUROPÄISCHE PATENTSCHRIFT

(11) **EP 1 862 115 B1**
(45) Veröffentlichungstag und Bekanntmachung des Hinweises auf die Patenterteilung: **18.03.2009**
(21) Anmeldenummer: 06011219.0
(22) Anmeldetag: 31.05.2006
(51) Int. Cl.: A61B 5/107

(54) **Registrierung mittels Strahlungsmarkierungselementen**
Registration with emitting marking elements
Enregistrement avec éléments de marquage à émission

(43) Veröffentlichungstag der Anmeldung: 05.12.2007
(73) Patentinhaber: BrainLAB AG, 85622 Feldkirchen (DE)
(72) Erfinder: Schmidt, Robert, 81371 München (DE)
(74) Vertreter: Gassenhuber, Andreas

(56) Entgegenhaltungen:
- EP-A- 1 302 172
- DE-A1- 4 417 872
- US-A- 5 848 967
- US-A1- 2002 087 101
- US-A1- 2005 182 461

## Beschreibung

Die Erfindung betrifft ein Verfahren und eine Vorrichtung zum Registrieren zumindest eines Teils eines Objekts, insbesondere eines Körpers oder Körperteils eines Patienten, zur Verwendung in einem medizinischen Navigationssystem, wobei auf die Oberfläche des Objekts eine Strahlungsmarkierungselemente, wie Quantenpunkte, enthaltende Substanz aufgetragen wird, wobei die Strahlungsmarkierungselemente von mindestens einer Kamera erfasst werden und basierend auf den erfassten Strahlungsmarkierungselementen eine Registrierung des Objekts durchgeführt werden kann, so dass nach dem Registrierungsvorgang Navigationsvorgänge bezüglich des Objekts mittels eines medizinischen Navigationssystems durchgeführt werden können.

Bekannte Verfahren und Vorrichtungen zur Registrierung oder Oberflächenregistrierung eines Objekts basieren auf Laserabtastung oder mechanischer Abtastung.

Mit einem so genannten Z-Touch-System oder einem mit Medtronic Fazer wird die Oberfläche eines Objekts mittels eines Lasers abgetastet und anschließend registriert.

Mit einem so genannten Softouch-System wird die Oberfläche eines Objekts mittels mechanischer Berührungen abgetastet und anschließend registriert.

Diese Verfahren sind langwierig und kompliziert und erfordern zusätzliche Hardware-Komponenten, wie das Z-Touch oder Softouch.

Aus der US 5,848,967 ist ein stereotaktisches System bekannt, wobei eine Sonde an den Kopf eines Patienten angelegt wird und das Verhältnis dieser Sonde zu der Anatomie des Patientenkopfes visualisiert wird, wobei an der Sonde angebrachte LED-Lichtquellen verwendet werden, die von Kameras zur optischen Registrierung der Sondenpositionen erfasst werden.

Es ist eine Aufgabe der vorliegenden Erfindung die bestehenden Nachteile aus dem Stand der Technik zu überwinden. Es ist weiter eine Aufgabe der vorliegenden Erfindung ein Verfahren und eine Vorrichtung zum einfachen und schnellen Registrieren eines Objekts bereitzustellen, welche keine zusätzlichen Hardware-Komponenten erfordern.

Diese Aufgabe wird durch den Gegenstand der unabhängigen Ansprüche gelöst. Vorteilhafte Ausführungsformen ergeben sich aus den abhängigen Ansprüchen.

Bei dem Verfahren zum Registrieren zumindest eines Teils eines Objekts, wie eines Körpers oder eines Körperteils eines Patienten, wird zunächst eine Substanz zumindest auf einen Teil der Oberfläche des Objekts, wie einen Arm oder ein Bein oder ein anderes Körperteil oder einen anderen Teil des Körpers eines Patienten, aufgebracht oder aufgetragen. Die aufgetragene Substanz, welche eine Creme oder Paste sein kann, enthält eine Vielzahl von Strahlungsmarkierungselementen, wie Quantenpunkte, welche durch das Aufbringen der Substanz auf dem Objekt nahezu gleichmäßig verteilt werden. Die Strahlungsmarkierungselemente emittieren Strahlung, insbesondere im IR-Bereich, oder können in einen oder mehrere Zustände angeregt werden, in denen sie Strahlung emittieren.

Ist die Substanz zumindest auf einem Teil der Oberfläche des Objekts oder des Körperteils aufgetragen oder aufgebracht und sind die in der Substanz enthaltenen Strahlungsmarkierungselemente auf der Oberfläche des Objekts verteilt so kann die Vielzahl von Strahlungsmarkierungselementen oder so kann zumindest ein Teil der Vielzahl von Strahlungsmarkierungselementen mittels einer Kamera, insbesondere einer IR-Kamera, erfasst werden, in dem die von den Strahlungsmarkierungselementen emittierte Strahlung von der Kamera detektiert oder erfasst wird.

Beispielsweise kann eine Kamera so ausgebildet und/oder angeordnet sein, dass sie in einer ersten Position und/oder Orientierung relativ zu dem Objekt oder Körperteil die Vielzahl von Strahlungsmarkierungselementen erfassen kann und in eine von der ersten Position und/oder Orientierung verschiedene zweite Position und/oder Orientierung relativ zu dem Objekt bewegt werden kann, in welcher sie wieder die Vielzahl von Strahlungsmarkierung erfassen kann.

Auch kann die Kamera so angeordnet und/oder ausgebildet sein, dass sie in mindestens einer ersten Position und/oder Orientierung relativ zu dem Objekt oder Körperteil zumindest einen Teil der Vielzahl von Strahlungsmarkierungen erfasst und in mindestens einer zweiten von der ersten Position und/oder Orientierung verschiedenen Position und/oder Orientierung relativ zu dem Objekt zumindest die Strahlungsmarkierungselemente erfasst, die in der ersten Position und/oder Orientierung der Kamera nicht von der Kamera erfasst werden. Insbesondere kann die Kamera relativ zu dem Objekt oder Körperteil beweglich ausgebildet sein und kann aus der mindestens einer ersten Position und/oder Orientierung in die mindestens eine zweite Position und/oder Orientierung relativ zum Objekt bewegt werden. Vorzugsweise ist die Kamera so ausgebildet, dass sie zumindest um einen Teil des Objekts oder vollständig um das Objekt bewegt werden kann, um die Vielzahl von Strahlungsmarkierungen auf der Oberfläche des Objekts zu erfassen.

Beispielsweise können auch zwei Kameras verwendet werden, wobei eine erste Kamera an einer ersten festen oder veränderlichen Position und/oder Orientierung und eine zweite Kamera an einer von der ersten Position und/oder Orientierung verschiedenen zweiten festen oder veränderlichen Position und/oder Orientierung relativ zu dem Objekt oder Körperteil positioniert sein können. In der ersten Position und/oder Orientierung der ersten Kamera kann beispielsweise ein Teil oder können alle der Strahlungsmarkierungselemente auf der Oberfläche des Objekts von der ersten Kamera erfasst werden. In der zweiten Position und/oder Orientierung der zweiten Kamera kann ein Teil oder können alle der Strahlungsmarkierungselemente von der zweiten Kamera erfasst werden, wobei vorzugsweise zumindest die Strahlungsmarkierungselemente von der zweiten Kamera erfasst werden, welche nicht von der ersten Kamera in der ersten Position und/oder Orientierung erfasst werden.

Vorzugsweise kann eine Kamera oder können zwei, drei, vier, fünf, sechs oder mehr Kameras verwendet werden.

Die Position und/oder Orientierung oder die Positionen und/oder Orientierungen der Kamera bzw. der Kameras relativ zu einem Referenzkoordinatensystem, wie einem feststehenden oder unveränderlichen Weltkoordinatensystem, sind bekannt oder ermittelbar. Zum Beispiel kann die Kamera oder können die Kameras in eine oder mehrere vorgegebene oder bekannte Positionen und/oder Orientierungen gebracht werden, in denen sie die Strahlungsmarkierungselemente erfassen oder die Strahlung der Strahlungsmarkierungselemente erfassen. Auch kann an der mindestens einen Kamera eine Trackingreferenz oder ein Referenzstern angeordnet sein, welcher zum Beispiel von einem Trackingsystem erfasst werden kann, so dass mittels des Trackingsystems die Position und/oder Orientierung des Referenzsterns und damit die Position und/oder Orientierung der mindestens einen Kamera relativ zu dem Referenz- oder Weltkoordinatensystem ermittelt werden kann.

Beispielsweise kann die Position und/oder Orientierung der mindestens einen Kamera kontinuierlich unabhängig von der jeweiligen Position ermittelt werden. Vorzugsweise wird die Position und/oder Orientierung der mindestens einen Kamera in der Position und/oder Orientierung ermittelt, in der sie das Objekt oder Körperteil zur Erfassung der Strahlungsmarkierungselemente abtastet oder aufnimmt.

Unter Berücksichtigung der Position und/oder Orientierung der mindestens einen Kamera relativ zu dem Referenzkoordinatensystem kann aus den erfassten Strahlungsmarkierungselementen die dreidimensionale Raumposition der Vielzahl von Strahlungsmarkierungselementen relativ zu dem Referenzkoordinatensystem ermittelt werden. Damit ist die Position der Strahlungsmarkierungen auf der Oberfläche des Objekts in einem Weltkoordinatensystem bekannt und das Objekt oder die Oberfläche des Objekts oder Körperteils kann basierend auf den dreidimensionalen Raumpositionen der Vielzahl von Strahlungsmarkierungselementen registriert werden.

Insbesondere kann die Vielzahl von Strahlungsmarkierungselementen aus mindestens zwei unterschiedlichen Positionen und/oder Orientierungen von der mindestens einen Kamera detektiert werden, wobei beispielsweise eine Kamera in verschiedene Positionen und/oder Orientierungen bewegt werden kann oder mehrere Kameras in verschiedenen Positionen und/oder Orientierungen feststehend oder beweglich relativ zu dem Objekt oder Körperteil positioniert oder angeordnet sein können.

Als Substanz kann eine Farbe, eine Flüssigkeit, eine Creme, oder eine Paste verwendet werden, welche auf die Oberfläche des Objekts, wie des Körperteils des Patienten, insbesondere einem Gesicht oder dem Oberkörper oder einem Arm oder Bein eines Patienten, insbesondere gleichmäßig, auftragbar ist. Die Strahlungsmarkierungselemente sind mit der Substanz vorzugsweise gleichmäßig vermischt oder in die Substanz gleichmäßig eingemischt, so dass nach einem Aufbringen der Substanz auf dem Objekt oder dem Körperteil des Patienten die Strahlungsmarkierungen über einen Großteil oder einen großen Teil des Körperteils oder über das gesamte Körperteil vorzugsweise zumindest nahezu gleichmäßig verteilt sind.

Die Strahlungsmarkierungselemente können sich in einem Grundzustand befinden und in dem Grundzustand mit der Substanz, wie der Paste oder Creme, gemischt werden und auf das Objekt oder Körperteil aufgetragen werden. Auch können die Strahlungsmarkierungselemente in einem angeregten Zustand mit der Substanz gemischt werden und mit der Substanz auf der Oberfläche des Objekts oder des Körperteils aufgebracht werden, so dass die Strahlungsmarkierungselemente durch Übergang in einen weiteren Zustand, wie den Grundzustand, Strahlung, insbesondere IR-Strahlung, emittieren können, welche von der mindestens einen Kamera erfasst werden kann.

Bevorzugt können als Strahlungsmarkierungselemente nanoskopische Strukturen, zum Beispiel aus Halbleitermaterial, wie Quantenpunkte, verwendet werden, deren Ladungsträger räumlich in allen drei Raumdimensionen soweit eingeschränkt sind, dass ihre Energie nicht mehr kontinuierliche sondern nur noch diskrete Werte annehmen kann. Vorzugsweise wird eine Substanz zumindest auf einen Teil des Objekts oder des Körpers oder des Körperteils eines Patienten aufgebracht, welche eine Vielzahl von Quantenpunkten enthält, welche zum Beispiel mittels einer externen Strahlungsquelle angeregt werden können und Strahlung, insbesondere IR-Strahlung, emittieren können. Als Quantenpunkte können insbesondere Quantenpunkte aus Halbleitermaterial, wie aus lnGaAs, CdSe, GaInP oder InP verwendet werden.

Die erfindungsgemäße Vorrichtung zum Registrieren zumindest eines Teils eines Objekts, insbesondere eines Körpers oder Körperteiles eines Patienten, umfasst mindestens eine Kamera, insbesondere mindestens eine IR-Kamera, und eine Recheneinheit, wie einen Computer, welche mit der mindestens einen Kamera drahtlos oder drahtgebunden verbunden ist. Die Strahlungsmarkierungselemente einer auf dem Objekt oder dem Körperteil, insbesondere nahezu gleichmäßig, aufgebrachten Substanz, welche eine Vielzahl von Strahlungsmarkierungselementen enthält, können von der mindestens einen Kamera erfasst werden. Die mindestens eine Kamera ist so angeordnet und/oder ausgebildet, dass sie die Strahlung, insbesondere die IR-Strahlung, zumindest eines Teils der Vielzahl von Strahlungsmarkierungselementen erfassen kann. Vorzugsweise umfasst die erfindungsgemäße Vorrichtung eine Kamera, welche relativ zu dem Objekt oder Körperteil beweglich ausgebildet ist.

Befindet sich die Kamera an einer ersten Position und/oder Orientierung relativ zu dem Objekt kann zumindest ein Teil der Vielzahl von Strahlungsmarkierungselementen von der Kamera erfasst werden. Die Kamera kann in mindestens eine weitere Position und/oder Orientierung relativ zu dem Objekt bewegt werden oder kann in mindestens einer weiteren Position und/oder Orientierung positioniert sein, in welcher sie zumindest einen weiteren Teil oder alle der Vielzahl von Strahlungsmarkierungselementen detektieren kann, insbesondere zumindest die Strahlungsmarkierungselemente detektieren kann, welche in der ersten Position nicht von der Kamera erfasst werden konnten.

Bevorzugt kann die Vorrichtung auch zwei oder mehr relativ zu dem Objekt feststehende oder bewegliche Kameras umfassen. Zum Beispiel kann eine erste Kamera in mindestens einer ersten festen oder veränderlichen Position und/oder Orientierung angeordnet sein, in der die Kamera zumindest einen Teil oder alle der Strahlungsmarkierungselemente erfassen kann. Die mindestens eine zweite Kamera kann beispielsweise in mindestens einer zweiten festen oder veränderlichen Position und/oder Orientierung angeordnet sein, in der sie zumindest einen zweiten Teil oder alle der Strahlungsmarkierungselemente erfassen kann, wobei vorzugsweise zumindest die Strahlungsmarkierungselemente detektiert werden, welche nicht in der mindestens einen ersten Position und/oder Orientierung detektiert werden.

Auch können mehr als zwei feste oder bewegliche Kameras verwendet werden, welche in ihren jeweiligen Positionen und/oder Orientierungen einen Teil oder alle Strahlungsmarkierungselemente erfassen können. Insbesondere können die beweglichen Kameras so ausgebildet sein, dass sie zumindest um einen Teil des Objekts oder um den Teil des Objekts oder Körperteils herumführbar sind, auf welchem sich die Substanz befindet.

Die Position und/oder Orientierung der mindestens einen Kamera kann bezüglich eines Referenzkoordinatensystems bekannt sein oder kann bezüglich eines Referenzkoordinatensystems ermittelt werden. Zum Beispiel kann die Position und/oder Orientierung der mindestens einen Kamera, wie die Position und/oder Orientierung der Kamera, in welcher sie die Strahlungsmarkierungselemente erfasst, dadurch bekannt sein, dass die Kamera in bekannte vorgegebene Positionen und/oder Orientierungen, insbesondere automatisch, bewegt wird. Diese Positionen und/oder Orientierungen können fest vorgegeben sein oder können zum Beispiel bei jedem Registrierungsvorgang neu berechnet werden. Zum Beispiel können Positionen und/oder Orientierungen mittels der Recheneinheit berechnet werden, in welcher möglichst viele Strahlungsmarkierungselemente erfasst werden können. Auch können Positionen und/oder Orientierungen errechnet werden, in denen möglichst wenige, wie keine, oder eine vorgegebene oder ideale Anzahl oder möglichst viele Strahlungsmarkierungselemente erneut erfasst werden.

Vorzugsweise kann die Recheneinheit Wunschpositionen ermitteln, in welchen oder in welche die mindestens eine Kamera positioniert sein bzw. werden kann, um mit möglichst wenigen Aufnahmen alle Strahlungsmarkierungselemente einmal, zweimal, dreimal oder viermal erfassen zu können. Insbesondere kann vorgegeben oder ermittelt werden, wie oft ein Strahlungsmarkierungselement erfasst werden muss, um seine dreidimensionale Raumposition ermitteln zu können.

Beispielsweise kann von der Recheneinheit eine erste Wunschposition der Kamera ermittelt werden, in der möglichst viele Strahlungsmarkierungselemente von der Kamera erfassbar sind. Auch kann von der Recheneinheit eine zweite Wunschposition der Kamera ermittelt, in der wieder möglichst viele Strahlungsmarkierungselemente von der Kamera erfasst werden. Auch kann von der Recheneinheit eine dritte Wunschposition der Kamera ermittelt werden, von der aus möglichst wenige Strahlungsmarkierungselemente zum dritten Male erfasst werden, von der aus aber zumindest die Strahlungsmarkierungselemente erfasst werden, welche nur einmal, wie in der ersten oder der zweiten Position der Kamera, von der Kamera erfasst wurden. Damit kann beispielsweise die Anzahl der nötigen Aufnahmen der Kamera minimiert und die erforderliche Zeit für die Registrierung minimiert werden.

Auch kann die Position und/oder Orientierung der Kamera dadurch ermittelt werden, dass an der Kamera eine Trackingreferenz oder ein Referenzstern angeordnet ist, deren bzw. dessen Position und/oder Orientierung relativ zu dem Referenzkoordinatensystem oder einem Weltkoordinatensystem von einem Trackingsystem erfasst und ermittelt werden kann, so dass auch die Position und/oder Orientierung der Kamera relativ zu dem Referenzkoordinatensystem oder Weltkoordinatensystem ermittelt werden kann.

Mittels der Recheneinheit können unter Berücksichtigung der ermittelten Position und/oder Orientierung der mindestens einen Kamera basierend auf den von der mindestens einen Kamera erfassten Strahlungsmarkierungselementen die dreidimensionalen Raumpositionen der Vielzahl von Strahlungsmarkierungselementen auf der Oberfläche des Objekts relativ zu dem Referenzkoordinatensystem oder Weltkoordinatensystem ermittelt werden. Vorzugsweise werden dabei die Strahlungsmarkierungselemente aus so vielen verschiedenen Positionen und/oder Orientierung der Kamera erfasst, dass alle oder nahezu alle Strahlungsmarkierungselemente mindestens einmal oder zweimal oder dreimal erfasst wurden oder dass eine Rekonstruktion der Oberfläche des Objekts oder des Körperteils basierend auf den erfassten Strahlungsmarkierungselementen möglich ist.

Die Recheneinheit kann die erfassten Strahlungsmarkierungselemente mit den ermittelten Positionen der Kamera, in denen die Strahlungsmarkierungselemente aufgenommen wurden, so in Bezug setzen, dass auf die dreidimensionale Raumposition der Strahlungsmarkierungselemente relativ zum dem Referenz- oder Weltkoordinatensystem geschlossen werden kann. Aus den dreidimensionalen Raumpositionen der Strahlungsmarkierungselemente kann auf die Oberfläche des Körperteils oder Objekts geschlossen werden und das Objekt oder das Körperteil oder die Oberfläche des Objekts oder Körperteils kann, zum Beispiel mit zuvor gemachten Aufnahmen des Objekts oder des Körperteils, registriert werden.

Weiter kann die erfindungsgemäße Vorrichtung eine Anregungsquelle umfassen, welche Strahlung einer vorgegebenen Frequenz oder eines vorgegebenen Frequenzspektrums abgeben kann. Die Strahlung der Anregungsquelle kann eine solche Frequenz aufweisen, dass sie die Strahlungsmarkierungselemente, wie die Quantenpunkte, in einen Zustand anregen kann, in welchem die Strahlungsmarkierungselemente oder Quantenpunkte eine Strahlung, insbesondere eine IR-Strahlung, emittieren oder abgeben, welche von der mindestens einen Kamera erfasst werden kann. Insbesondere kann die Anregungsquelle fest oder beweglich ausgebildet sein und kann beispielsweise an der mindestens einen Kamera angeordnet sein oder in die mindestens eine Kamera integriert sein.

Die Erfindung wird nachfolgend anhand eines bevorzugten Ausführungsbeispiels beschrieben werden. Es zeigt:

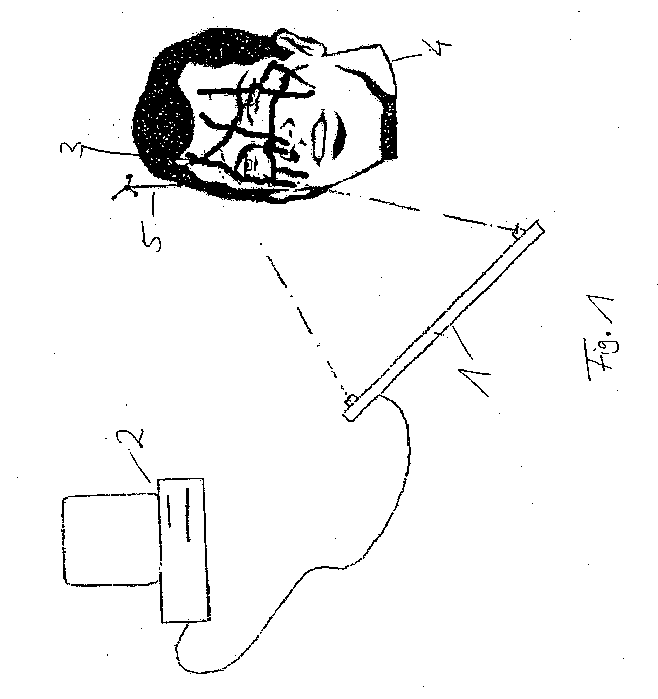

- Figur 1: eine Ausführungsform der vorliegenden Erfindung mit einer Kamera 1 und einer Recheneinheit 2 zum Registrieren eines Teils eines Gesichts eines Patienten.

Figur 1 zeigt eine Ausführungsform der vorliegenden Erfindung mit einer IR-Kamera 1, welche drahtgebunden mit einem Computer 2 verbunden ist. Auf einem Teil eines Gesichts 4 eines Patienten ist eine Creme, Farbe, Flüssigkeit oder Paste in einem vorgegebenen oder ermittelbaren Muster aufgebracht. Die Creme enthält eine Vielzahl von Quantenpunkten 3, welche in einem angeregten Zustand IR-Strahlung emittieren, welche von der IR-Kamera 1 erfasst werden kann. Die Position der IR-Kamera 1 im Raum relativ zu einem Referenz- oder Weltkoordinatensystem ist dem Computer 2 bekannt und die Position des Patienten 4 ist bekannt oder kann ermittelt werden, indem die Position und/oder Orientierung des an dem Kopf des Patienten angeordneten Referenzsterns 5 ermittelt wird. Die IR-Kamera 1 kann in einer ersten Position zumindest einen Teil oder alle Quantenpunkte 3 erfassen, indem sie die von den Quantenpunkten 3 abgegebene oder emittierte IR-Strahlung detektiert. Aus der Position der Kamera 1 relativ zu dem Referenzkoordinatensystem und aus der detektierten IR-Strahlung ermittelt der Computer 2 die dreidimensionale Raumposition der erfassten Quantenpunkte 3. Weiter kann die IR-Kamera 1 in eine weitere Position bewegt werden, in welcher sie einen weiteren Teil der Quantenpunkte 3 erfassen kann, so dass die dreidimensionale Raumposition der in der zweiten Position erfassten Quantenpunkte 3 von dem Computer 2 ermittelt werden kann. Bevorzugt werden die Quantenpunkte 3 aus mindestens zwei verschiedenen Positionen der IR-Kamera 1 erfasst, so dass aus den erfassten Strahlungen in den verschiedenen Aufnahmepositionen der IR-Kamera 1 auf die Position der Quantenpunkte 3 im Raum geschlossen werden kann. Ist ein Großteil oder sind alle der Positionen der Quantenpunkte 3 im Raum relativ zu dem Referenz- oder Weltkoordinatensystem bekannt, so kann aus den ermittelten Positionen der Quantenpunkte 3 von dem Computer 2 die Oberfläche des Gesichts 4 des Patienten rekonstruiert oder ermittelt werden. Die Oberfläche des Gesichts 4 des Patienten ist somit im dreidimensionalen Raum bekannt und kann zum Beispiel mit weiteren Patientendatensätzen des Patienten, wie präoperativ gemachten Aufnahmen oder mittels bildgebender Verfahren gemachten Aufnahmen des Patienten, registriert werden.

## Patentansprüche

1. Verfahren zum Registrieren zumindest eines Teils eines Objekts (4), insbesondere eines Körpers oder Körperteils eines Patienten, mit den folgenden Schritten:
- Aufbringen einer Substanz zumindest auf einen Teil der Oberfläche des Objekts (4), wobei die Substanz eine Vielzahl von Strahlungsmarkierungselementen (3) enthält, welche Strahlung, insbesondere IR-Strahlung, emittieren;
- Abtasten oder Aufnehmen der Oberfläche des Objekts (4) mit mindestens einer Kamera (1), insbesondere einer IR-Kamera, deren dreidimensionale Position und/oder Orientierung relativ zu einem Referenzkoordinatensystem bekannt oder ermittelbar ist, wobei die Kamera (1) so angeordnet und/oder ausgebildet ist, dass sie zumindest einen Teil der Vielzahl von Strahlungsmarkierungselementen (3) auf der Oberfläche des Objekts (4) erfassen kann;
- Ermitteln der dreidimensionalen Raumpositionen der Vielzahl von Strahlungsmarkierungselementen (3) auf der Oberfläche des Objekts (4) relativ zu dem Referenzkoordinatensystem basierend auf den von der Kamera (1) erfassten Strahlungsmarkierungselementen (3) und der dreidimensionalen Position und/oder Orientierung der Kamera (1) relativ zu dem Referenzkoordinatensystem; und
- Registrierung des Objekts (4) basierend auf den dreidimensionalen Raumpositionen der Vielzahl von Strahlungsmarkierungselementen (3),
- **dadurch gekennzeichnet, daß** als Substanz eine Farbe, eine Flüssigkeit, eine Creme oder eine Paste verwendet wird, welche auf die Oberfläche des Objekts (4) gleichmäßig, auftragbar ist.

2. Verfahren nach dem vorhergehenden Anspruch, wobei mindestens zwei Kameras (1) verwendet werden.

3. Verfahren nach einem der vorhergehenden Ansprüche, wobei zwei, drei, vier, fünf, sechs oder mehr Kameras (1) verwendet werden.

4. Verfahren nach einem der vorhergehenden Ansprüche, wobei als die mindestens eine Kamera (1) eine relativ zu dem Objekt (4) feststehende oder bewegliche Kamera (1) verwendet wird.

5. Verfahren nach dem vorhergehenden Anspruch, wobei die relativ zu dem Objekt (4) bewegliche Kamera (1) zur Erfassung zumindest eines Teils der Vielzahl von Strahlungsmarkierungselementen (3) um den Teil des Objekts (4) herumgeführt wird.

6. Verfahren nach einem der vorherigen Ansprüche, wobei die Vielzahl von Strahlungsmarkierungselementen (3) aus unterschiedlichen Positionen von der mindestens einen Kamera (1) detektiert wird.

7. Verfahren nach einem der vorhergehenden Ansprüche, wobei als Substanz eine Substanz verwendet wird, in der die Vielzahl von Strahlungsmarkierungselementen (3) gleichmäßig mit oder in der Substanz gemischt bzw. verteilt ist.

8. Verfahren nach einem der vorhergehenden Ansprüche, wobei die Strahlungsmarkierungselemente (3) in mindestens einem angeregten Zustand Strahlung, insbesondere IR-Strahlung, emittieren.

9. Verfahren nach einem der vorhergehenden Ansprüche, wobei als Substanz eine eine Vielzahl von Quantenpunkten enthaltende Substanz verwendet wird, wobei die Quantenpunkte als die Vielzahl von Strahlungsmarkierungselementen (3) in der Substanz von der mindestens einen Kamera (1) detektiert werden.

10. Verfahren nach dem vorhergehenden Anspruch, wobei als Quantenpunkte Quantenpunkte aus Halbleitermaterial, insbesondere aus InGaAs, CdSe, GaInP oder InP, verwendet werden.

11. Vorrichtung zum Registrieren zumindest eines Teils eines Objekts (4), insbesondere eines Körpers oder Körperteils eines Patienten mit:
- mindestens einer Kamera (1), insbesondere einer IR-Kamera, deren dreidimensionale Position und/oder Orientierung relativ zu einem Referenzkoordinatensystem bekannt oder ermittelbar ist, wobei die Kamera (1) so angeordnet und/oder ausgebildet ist, dass sie Strahlung, insbesondere IR-Strahlung, zumindest eines Teils einer Vielzahl von Strahlung emittierenden Strahlungsmarkierungselementen (3) detektieren kann, welche in einer auf das Objekt (4) aufgebrachten Substanz enthalten ist;
- einer Recheneinheit (2), welche mit der mindestens einen Kamera (1) drahtlos oder drahtgebunden verbunden ist, zum Ermitteln der dreidimensionalen Raumpositionen der Vielzahl von Strahlungsmarkierungselementen (3) auf der Oberfläche des Objekts (4) relativ zu einem Referenzkoordinatensystem basierend auf den von der mindestens einen Kamera (1) erfassten Strahlungsmarkierungselementen (3) und der dreidimensionalen Position und/oder Orientierung der Kamera (1) relativ zu dem Referenzkoordinatensystem und zum Registrieren des Objekts (4) basierend auf den dreidimensionalen Raumpositionen der Vielzahl von Strahlungsmarkierungselementen (3).
**dadurch gekennzeichnet, daß** die Substanz eine Farbe, eine Flüssigkeit, eine Creme oder eine Paste ist, welche auf die Oberfläche des Objekts (4) gleichmäßig auftragbar ist.

12. Vorrichtung nach dem vorhergehenden Anspruch mit mindestens zwei Kameras (1).

13. Vorrichtung nach einem der zwei vorhergehenden Ansprüche, wobei die mindestens eine Kamera (1) relativ zu dem Objekt (4) feststehend oder beweglich ist.

14. Vorrichtung nach dem vorhergehenden Anspruch, wobei die relativ zu dem Objekt (4) bewegliche Kamera (1) so ausgebildet ist, dass sie zur Erfassung zumindest eines Teils der Vielzahl von Strahlungsmarkierungselementen (3) um den Teil des Objekts (4) herumführbar ist.

15. Vorrichtung nach einem dem vier vorhergehenden Anspruch mit einem Trackingsystem zum Erfassen der dreidimensionalen Raumposition eines auf oder an der Kamera (1) angeordneten Referenzsterns.

16. Vorrichtung nach einem der fünf vorhergehenden Ansprüche mit einer Anregungsquelle, welche die Vielzahl von in der Substanz enthaltenen Strahlungsmarkierungselementen (3) in mindestens einen angeregten Zustand anregen kann, in dem die Vielzahl von Strahlungsmarkierungselementen (3) Strahlung, insbesondere IR-Strahlung, emittieren kann.

17. Vorrichtung nach dem vorhergehenden Anspruch, wobei die Anregungsquelle an der mindestens einen Kamera (1) angeordnet oder in der oder in die mindestens einen Kamera (1) enthalten bzw. integriert ist.

## Claims

1. A method for registering at least a part of an object (4), in particular a body or part of a patient's body, comprising the following steps:
- applying a substance to at least a part of the surface of the object (4), wherein the substance contains a plurality of radiation marking elements (3) which emit radiation, in particular infrared radiation;
- scanning or recording the surface of the object (4) using at least one camera (1), in particular an infrared camera, the three-dimensional position and/or orientation of which is known or can be ascertained relative to a reference co-ordinate system, wherein the camera (1) is arranged and/or configured such that it can detect at least a part of the plurality of radiation marking elements (3) on the surface of the object (4);
- ascertaining the three-dimensional spatial positions of the plurality of radiation marking elements (3) on the surface of the object (4) relative to the reference co-ordinate system, on the basis of the radiation marking elements (3) detected by the camera (1) and the three-dimensional position and/or orientation of the camera (1) relative to the reference co-ordinate system; and
- registering the object (4) on the basis of the three-dimensional spatial positions of the plurality of radiation marking elements (3),
**characterised in that** a paint, liquid, cream or paste which can be uniformly applied to the surface of the object (4) is used as the substance.

2. The method according to the preceding claim, wherein at least two cameras (1) are used.

3. The method according to any one of the preceding claims, wherein two, three, four, five, six or more cameras (1) are used.

4. The method according to any one of the preceding claims, wherein a camera (1) which is fixed or movable relative to the object (4) is used as the at least one camera (1).

5. The method according to the preceding claim, wherein the camera (1) which is movable relative to the object (4) is guided around at least a part of the object (4) in order to detect at least a part of the plurality of radiation marking elements (3).

6. The method according to any one of the preceding claims, wherein the plurality of radiation marking elements (3) are detected from different positions of the at least one camera (1).

7. The method according to any one of the preceding claims, wherein a substance in which the plurality of radiation marking elements (3) are uniformly mixed with or distributed in the substance is used as the substance.

8. The method according to any one of the preceding claims, wherein the radiation marking elements (3) emit radiation, in particular infrared radiation, in at least one excited state.

9. The method according to any one of the preceding claims, wherein a substance containing a plurality of quantum dots is used as the substance, wherein the quantum dots are detected by the at least one camera (1) as the plurality of radiation marking elements (3) in the substance.

10. The method according to the preceding claim, wherein quantum dots made of a semiconductor material, in particular InGaAs, CdSe, GaInP or InP, are used as the quantum dots.

11. A device for registering at least a part of an object (4), in particular a body or part of a patient's body, comprising:
- at least one camera (1), in particular an infrared camera, the three-dimensional position and/or orientation of which is known or can be ascertained relative to a reference co-ordinate system, wherein the camera (1) is arranged and/or configured such that it can detect radiation, in particular infrared radiation, of at least a part of a plurality of radiation-emitting radiation marking elements (3) contained in a substance applied to the object (4);
- a computational unit (2) which is connected wirelessly or via wiring to the at least one camera (1), for ascertaining the three-dimensional spatial positions of the plurality of radiation marking elements (3) on the surface of the object (4) relative to a reference co-ordinate system, on the basis of the radiation marking elements (3) detected by the at least one camera (1) and the three-dimensional position and/or orientation of the camera (1) relative to the reference co-ordinate system, and for registering the object (4) on the basis of the three-dimensional spatial positions of the plurality of radiation marking elements (3),
**characterised in that** the substance is a paint, liquid, cream or paste which can be uniformly applied to the surface of the object (4).

12. The device according to the preceding claim, comprising at least two cameras (1).

13. The device according to any one of the preceding two claims, wherein the at least one camera (1) is fixed or movable relative to the object (4).

14. The device according to the preceding claim, wherein the camera (1) which is movable relative to the object (4) is configured such that it can be guided around at least a part of the object (4) in order to detect at least a part of the plurality of radiation marking elements (3).

15. The device according to any one of the preceding four claims, comprising a tracking system for detecting the three-dimensional spatial position of a reference star arranged on the camera (1).

16. The device according to any one of the preceding five claims, comprising an excitation source which can excite the plurality of radiation marking elements (3) contained in the substance into at least one excited state in which the plurality of radiation marking elements (3) can emit radiation, in particular infrared radiation.

17. The device according to the preceding claim, wherein the excitation source is arranged on the at least one camera (1) or contained in or integrated into the at least one camera (1).

## Revendications

1. Procédé de repérage d'au moins une partie d'un objet (4), en particulier du corps ou d'une partie corporelle d'un patient, comportant les étapes suivantes .
- appliquer une substance sur au moins une partie de la surface de l'objet (4), la substance contenant un grand nombre d'éléments de repérage à émission (3) qui émettent un rayonnement, en particulier un rayonnement IR ;
- balayer ou réaliser une prise de vue de la surface de l'objet (4) avec au moins une caméra (1), en particulier une caméra IR, dont la position et/ou l'orientation tridimensionnelles par rapport à un système de coordonnées de référence est connue ou peut être déterminée, la caméra (1) étant disposée et/ou conçue de manière à pouvoir relever au moins une partie du grand nombre d'éléments de repérage à émission (3) sur la surface de l'objet (4) ;
- déterminer les positions spatiales tridimensionnelles du grand nombre d'éléments de repérage à émission (3) sur la surface de l'objet (4) par rapport au système de coordonnées de référence sur la base des éléments de repérage à émission (3) relevés par la caméra (1) et de la position et/ou orientation tridimensionnelles de la caméra (1) par rapport au système de coordonnées de référence ; et
- repérer l'objet (4) sur la base des positions spatiales tridimensionnelles du grand nombre d'éléments de repérage à émission (3),
**caractérisé en ce qu'**on utilise comme substance une encre, un liquide, une crème ou une pâte qui peut être appliquée uniformément sur la surface de l'objet (4).

2. Procédé selon la revendication 1, dans lequel on utilise au moins deux caméras (1).

3. Procédé selon l'une quelconque des revendications 1 ou 2, dans lequel on utilise deux, trois, quatre, cinq, six caméras (1) ou plus.

4. Procédé selon l'une quelconque des revendications 1 à 3, dans lequel on utilise une caméra (1) fixe ou mobile par rapport à l'objet (4), en tant que la au moins une caméra (1).

5. Procédé selon la revendication 4, dans lequel la caméra (1), mobile par rapport à l'objet (4), est guidée autour de la partie de l'objet (4), pour relever au moins une partie du grand nombre d'éléments de repérage à émission (3).

6. Procédé selon l'une quelconque des revendications 1 à 5, dans lequel on détecte le grand nombre d'éléments de repérage à émission (3) à partir de différentes positions de la au moins une caméra (1).

7. Procédé selon l'une quelconque des revendications 1 à 6, dans lequel on utilise comme substance une substance dans laquelle on mélange uniformément à la substance ou on répartit uniformément dans la substance le grand nombre d'éléments de repérage à émission (3).

8. Procédé selon l'une quelconque des revendications 1 à 7, dans lequel les éléments de repérage à émission (3) émettent un rayonnement, en particulier un rayonnement IR, dans au moins un état excité.

9. Procédé selon l'une quelconque des revendications 1 à 8, dans lequel on utilise comme substance une substance contenant un grand nombre de points quantiques, les points quantiques étant détectés par la au moins une caméra (1), en tant que grand nombre d'éléments de repérage à émission (3) dans la substance.

10. Procédé selon la revendication 9, dans lequel on utilise comme points quantiques, des points quantiques en un matériau semi-conducteur, en particulier en InGaAs, CdSe, GaInP ou InP.

11. Dispositif pour repérer au moins une partie d'un objet (4), en particulier du corps ou d'une partie corporelle d'un patient comportant :
- au moins une caméra (1), en particulier une caméra IR, dont la position et/ou l'orientation tridimensionnelles par rapport à un système de coordonnées de référence est connue ou peut être déterminée, la caméra (1) étant disposée et/ou conçue de manière à pouvoir détecter le rayonnement, en particulier le rayonnement IR, d'au moins une partie d'un grand nombre d'éléments de repérage à émission (3) émettant un rayonnement, qui sont contenus dans une substance appliquée sur l'objet (4) ;
- une unité informatique (2) qui est reliée sans fil ou par fil à la au moins une caméra (1), pour déterminer les positions spatiales tridimensionnelles du grand nombre d'éléments de repérage à émission (3) sur la surface de l'objet (4) par rapport à un système de coordonnées de référence, sur la base des éléments de repérage à émission (3) relevés par la au moins une caméra (1), et la position et/ou l'orientation tridimensionnelles de la caméra (1) par rapport au système de coordonnées de référence et pour repérer l'objet (4) sur la base des positions spatiales tridimensionnelles du grand nombre d'éléments de repérage à émission (3),
**caractérisé en ce que** la substance est une encre, un liquide, une crème ou une pâte qui est uniformément appliquée sur la surface de l'objet (4).

12. Dispositif selon la revendication 11 avec au moins deux caméras (1).

13. Dispositif selon l'une quelconque des revendications 11 ou 12, dans lequel la au moins une caméra (1) est fixe ou mobile par rapport à l'objet (4).

14. Dispositif selon la revendication 13, dans lequel la caméra (1), mobile par rapport à l'objet (4), est conçue de manière à pouvoir être guidée autour de la partie de l'objet (4), pour relever au moins une partie du grand nombre d'éléments de repérage à émission (3).

15. Dispositif selon l'une quelconque des revendications 11 à 14 avec un système de poursuite pour relever la position spatiale tridimensionnelle d'une étoile de référence disposée sur ou contre la caméra (1).

16. Dispositif selon l'une quelconque des revendications 11 à 15 avec une source d'excitation qui peut exciter le grand nombre d'éléments de repérage à émission (3) contenus dans la substance, dans au moins un état excité dans lequel le grand nombre d'éléments de repérage à émission (3) peut émettre un rayonnement, en particulier un rayonnement IR.

17. Dispositif selon la revendication 16, dans lequel la source d'excitation est disposée sur la au moins une caméra (1) ou contenue ou intégrée dans la ou dans la au moins une caméra (1).
